# EUROPÄISCHE PATENTSCHRIFT

(11) **EP 1 610 827 B1**
(45) Veröffentlichungstag und Bekanntmachung des Hinweises auf die Patenterteilung: **28.03.2007**
(21) Anmeldenummer: 04726440.3
(22) Anmeldetag: 08.04.2004
(51) Int. Cl.: A61L 2/28, G01N 31/22

(54) **STERILISATIONSTESTVORRICHTUNG**
STERILIZATION TESTING DEVICE
DISPOSITIF D'ESSAI DE STERILISATION

(30) Priorität: 10.04.2003 DE 10316690; 03.02.2004 DE 102004005377
(43) Veröffentlichungstag der Anmeldung: 04.01.2006
(73) Patentinhaber: Kretschmann, Harald, 35463 Fernwald (DE); Rauch, Andreas, 35423 Lich (DE)
(72) Erfinder: Kretschmann, Harald, 35463 Fernwald (DE); Rauch, Andreas, 35423 Lich (DE)
(74) Vertreter: Creutz, Dieter
(86) Internationale Anmeldenummer: PCT/DE2004/000758
(87) Internationale Veröffentlichungsnummer: WO 2004/091672

(56) Entgegenhaltungen:
- EP-A- 0 069 037
- EP-A- 1 308 175
- WO-A-01/56618
- DE-A- 10 213 066
- US-A- 4 596 696

## Beschreibung

Die Erfindung betrifft eine Sterilisationstestvorrichtung, aus mindestens zwei Gehäuseteilen, einer Testkammer mit Indikator, mit Öffnungen und Zuleitungen zur Zuführung des Sterilisationsmediums.

Sterilisationstestvorrichtungen werden genutzt, um nach einer Sterilisation mit ausreichender Sicherheit entscheiden zu können, ob der Sterilisationsvorgang erfolgreich war.

Als Sterilisationsmittel wird bevorzugt ein gas- oder dampfförmiges Medium vorgesehen z.B. Wasserdampf, Ethylenoxid oder Formaldehyd. Speziell bei sog. Dampfsterilisatoren, wie sie im Krankenhaus eingesetzt werden, muss realisiert werden, dass auch lange Schläuche sowie medizinische Werkzeuge und Geräte mit an Sicherheit grenzender Wahrscheinlichkeit sterilisiert werden. In der Regel wird bei Dampfsterilisatoren, vor dem Einlassen des z.B. üblicherweise 121°C oder 134°C heißen Sattdampfes, der Dampfsterilisator evakuiert. Dies bewirkt, dass das Sterilisationsmittel ungehindert durch im Sterilisationsgut verweilenden Restgase an die zu sterilisierenden Stellen gelangt. Hierzu ist es erforderlich, dass die Testkammer mit eingelegtem Indikator nur durch eine im Verhältnis zum Querschnitt sehr lange Zuleitung mit der Atmosphäre des Dampfsterilisator verbunden ist.

Die Ankopplung der Zuleitung an die Testkammer stellt den Schwachpunkt der Sterilisationstestvorrichtung dar.

So wird nach der DE 43 19 397 C1 festgestellt, dass unter den vorgenannten Bedingungen Beschädigungen der Zuleitung denkbar sind. Die Beschädigung kann an der Verbindung bzw. Kupplung zwischen Zuleitung und Testkammer, beginnen. Schon geringe Undichtigkeiten können ausreichen, dass der Weg der umgebenden Sterilisationsatmosphäre - unter Umgehung des langen Wegs durch die Zuleitung - abkürzt, so dass der in der Testkammer befindliche Indikator fälschlicherweise eine vollständige Sterilisation bzw. ausreichende Sterilisationsbedingungen anzeigt.

Die Verbindung der Zuleitung an die Testskammer wird vielfach auch deswegen als Schwachpunkt des Testsystems ausgewiesen, weil die Zuleitung lose an der Testkammer bzw. deren Behälter hängt und bei Anwendung des Prüfkörpers diese Kuppelstelle mechanisch belastet wird.

Nach der DE 43 19 397 C1 wird aus diesen Gründen die als Schlauch ausgebildete Zuleitung durch einen Pfropfen aus porösen Material ersetzt, der das Einströmen des Sterilisationsmittels in die Testkammer, wie eine Labyrinthdichtung erschwert.
Bei häufiger Benutzung hat diese Lösung dem Nachteil, dass der jeweilige Pfropfen nicht nur wie ein Labyrinth sondern wie eine Sperre gegenüber dem Sterilisationsmittel wirkt.

Mit der DE 197 24 158 A1 sollten die beschriebenen Nachteile dadurch gelöst werden, dass eine Sterilisationstestvorrichtungen mit einem als lang gestreckten Schlauch ausgebildeten Zuleitungsmittel geschaffen wird, wobei die Zuleitung in enger Packung, mechanisch unbeweglich, auf dem Behälter festgelegt ist.
Nachteil dieser Lösung ist allerdings der Umstand, dass die Zuleitung, wenn auch auf dem Behälter der Testkammer praktische fest verbundenen und damit die Anschlussstelle keiner mechanischen Belastung mehr unterliegt, diese dennoch vor Beschädigungen nicht geschützt ist. Zudem ist die Herstellung der vorgeschlagenen Lösung mit größerem Aufwand verbunden.

Die bekannten Sterilisationstestvorrichtungen weisen einen weiteren Nachteil auf.

Der Indikator muss vor dem Sterilisationsvorgang vom Bedienpersonal in den Sterilisator eingelegt und nach der Sterilisation zu Dokumentationszwecken wieder entfernt werden. In der Regel ist die Testkammer durch einen steckbaren oder schraubbaren Deckel verschlossen.

Diese Deckel weisen in der Regel eine Dichtung auf, die den Kurzschluss vom umgebenden Sterilisationsmedium und der Testkammer verhindern soll und regelmäßig geprüft und gegebenenfalls ersetzt werden muss. Weist der Deckel bzw. die Dichtung ein Leck auf, kann nicht nachgeprüft werden, ob der Indikator aufgrund der Undichtigkeit des Deckels oder der Dichtung oder aufgrund einer korrekten Sterilisation die erfolgreiche Sterilisation anzeigt.
Ebenfalls möglich ist, dass der Indikator beim Verschrauben oder Aufstecken des Deckels zwischen Testkammerwand und Deckel gequetscht wird.

Bei Sterilisationstestvorrichtungen ist es erforderlich, dass ein Indikator nur durch eine im Verhältnis zum Querschnitt sehr lange Zuleitung mit der Atmosphäre des Sterilisators verbunden ist. Durch die Wechselwirkung des Sterilisationsmediums, bei im Verhältnis zum Querschnitt sehr langer Zuleitung, mit dem Indikator lässt sich modellhaft nachweisen, dass auch Hohlräume sowie Schläuche oder poröse Materialien mit dem Sterilisationsmedium beaufschlagt bzw. durchdrungen werden.

In der DE 102 13 066 A1 wird eine Lösung für eine solche Sterilisationstestvorrichtung vorgeschlagen, bei der aus zwei dünnen Folien (Wandelementen) durch eine Ausstülpung, zumindest in der einen Folie, ein Diffusionshohlraum gebildet wird und die zweite Folie diesen Diffusionshohlraum nach außen hin sterilisationsmediendicht abschließt.
In dem Diffusionshohlraum ist ein Indikator angeordnet.
Diese Lösungen bietet eine Reihe von Vorteilen gegenüber den bestehenden Systemen. Zum Einen entfällt die Kuppelstelle von Testkammer zur Zuleitung und zum Zweiten ist es als Einwegartikel ausgelegt, was die Handhabung des Systems bedeutend vereinfacht.

Der Nachteil dieser Lösung ist, dass der Zuleitungsschlauch , hier Diffusionshohlraum genannt, ungeschützt in der Sterilisationskammer mechanischen Einflüssen ausgesetzt ist. Eine noch so kleine Porosität des Diffusionshohlraumes bewirkt, dass das Sterilisationsmedium unter Umgehung des kompletten Wegs über die gesamte Diffusionsstrecke an den Indikator gelangt.
Ein weiterer Nachteil ist, dass der Indikator nicht aus dem Testkörper entnommen werden kann. In der DE 102 13 066 A 1 ist zwar beschrieben, dass das komplette System archiviert werden soll, doch nach den Abmaßen wie sie in der prEN13060: 2002 bzw. in der EN 867-5 4.5 vorgeschlagen werden, ist das Prüfsystem mehrere Millimeter dick, so dass das Prüfsystem sich nicht zur kompletten Dokumentation eignet.

Eine weitere Lösung wird in der EP -A- 0069037 vorgeschlagen. Bei dieser deckt eine durchsichtige Folie mit oder ohne einem Trägerplättchen den Indikator vollständig ab. Diese ist in ihrem Aufbau und Struktur so ausgestaltet, dass sie selbst für das Sterilisationsmedium durchlässig ist und allein über diesen Weg das Sterilisationsmedium den Indikator erreichen kann.

Das zu prüfende Sterilisationsmedium gelangt hierbei nicht über Öffnungen und entsprechende Zuleitungen zum Indikator sondern ausschließlich über die durchlässige Folie.

Mit der WO- 01-56618 wird eine Lösung für eine Sterilisationstestvorrichtung beschrieben, bei der diese aus mindestens einem Paar von miteinander verbindbaren Körpern, zum einen aus einem im wesentlichen porösen Element, besteht, das eine Dampfpenetration zulässt und einem zweiten Körper, der aus einer Kammer besteht, die selbst sich wiederum aus drei Teilen zusammensetzt. Erfindungsnotwendigerweise wird das Sterilisationsmedium zunächst über das poröse Element eingeleitet, welches eine oder mehrere externe Oberflächen aufweist, durch die der Dampf aus vielerlei Richtungen aufgenommen werden kann. Erst von dort gelangt es in eine Kammer, die ein " gewundenes" Mittelpfadsystem aufweist. Dieser Teil des Gerätes ist durch einen Verschluss in zwei Teilkammern unterteilt, wobei der Dampf damit zunächst das Pfadsystem der einen Teilkammer durchlaufen muss, ehe er über einen Durchlass im Verschluss in die zweite Kammer eindringen und diese durchlaufen kann. Erst danach gelangt der Dampf, nachdem er diese Kammer wieder verlassen hat in einen weiteren separaten Körper, indem sich der Indikator befindet.

Der Erfindung liegt daher die Aufgabe zu Grunde, eine Sterilisationstestvorrichtung zu schaffen, deren Zuleitung zur Testkammer die für eine übliche Dampfsterilisation erforderlichen Abmessungen hat, ohne die Schwachpunkte an der Kupplungsstelle aufzuweisen und gleichzeitig geschützt vor mechanischen Einwirkungen zu sein. Die Sterilisationstestvorrichtung soll zudem neben ihrer hohen Kompaktheit es problemlos ermöglichen ein Öffnen und Schließen der Sterilisationstestvorrichtung zum Zweck der Entnahme des Indikators zu realisieren. Dabei soll sie fertigungstechnisch sehr einfach herzustellen sein, die Leistungsfähigkeit eines Sterilisators in einfacher Art bewerten lassen, sowie die Rückführbarkeit bei Einwegartikeln gewährleisten.

Diese Aufgabe wird erfindungsgemäß dadurch gelöst, dass die Sterilisationstestvorrichtung aus mindestens zwei aufeinander abgestimmten Gehäuseteilen (3) und (5) besteht, wobei in dem Gehäuseteil (3) ein Zuleitungsgraben (4) eingearbeitet ist, der zur Zuführung des Sterilisationsmediums dient.

Der Zuleitungsgraben (4) ist dabei so angeordnet, dass er den Grundkörper der Testkammer (1) bildet. Die Testkammer (1) nimmt den Indikator (2) auf, der den Abschluss des Sterilisationsvorganges anzeigt.
Der Zuleitungsgraben (4) weist sowohl zur Testkammer (1) als auch nach außen hin eine Öffnung (7)bzw. (7') auf. Damit kann von außen, über den dort offenen Zuleitungsgraben (4) das Sterilisationsmedium eingeleitet werden. Der Zuleitungsgraben (4) endet ebenfalls offen in die Testkammer (1). Anschlüsse und Kupplungsstellen sind nicht erforderlich.

Der in das Gehäuseteil (3) eingearbeitete Zuleitungsgraben (4) weist einen beliebigen Querschnitt auf. Vorzugsweise kann dieser rechteckig oder quadratisch ausgebildet werden. Da der Zuleitungsgraben (4) einen sehr dünnen Querschnitt hat, bei gleichzeitig im Verhältnis dazu erheblicher Länge, macht es besonderen Sinn einen solchen Zuleitungsgraben (4) in Mäander- oder Schneckenform auszubilden.

Grundsätzlich können wahlweise die Gehäuseteile so gestaltet sein, dass sie die Möglichkeit bieten, sich sowohl vor als auch nach dem Sterilisationsvorgang öffnen und schließen zu lassen, um an den Indikator (2) zu gelangen oder die Gehäuseteile (3) und (5) sind fest miteinander verbunden.
Letztere Variante stellt eine besonders vorteilhafte Ausgestaltung der Erfindung dar. In diesem Fall wird der Indikator (2) in der Testkammer (1) bereits bei der Herstellung der Sterilisationstestvorrichtung eingelegt, so dass ein Öffnen der Testvorrichtung erst nach Abschluss des Sterilisationsvorganges zum Entnehmen des Indikators (2) erforderlich ist.

Um den Zugang zur Testkammer (1) zum Herausnehmen des Indikators (2) ermöglichen zu können, ist für die Fälle, bei denen die Gehäuseteile (3) und (5) fest miteinander verbunden sind, in dem Gehäuseteil (3) oder (5) eine Öffnung (6) vorgesehen, die mittels eines Verschlusses (12) verschlossen wird.

Eine solcher Verschlusses (12) kann in besonders vorteilhafter Weise als Einwegöffnung ausgebildet sein. Dies ist immer dann sinnvoll, wenn die beiden Gehäuseteile (3) und (5) fest miteinander verbunden sind und damit ebenfalls für den Einweggebrauch vorgesehen sind.

Die Erfindung lässt allerdings weitere Möglichkeiten zu, um bei Einweggebrauch oder Mehrweggebrauch der Sterilisationstestvorrichtung den Indikator (2) auf seine Reaktion prüfen zu können.

So sind in einer weiteren Ausführungsform der Sterilisationstestvorrichtung die Gehäuseteile (3) und (5) lösbar miteinander verbunden.
Bei einer solchen Ausführungsform lassen sich die Gehäuseteile (3) und (5) zum Einlegen und oder zum Entnehmen des Indikators (2) auf einfache Art öffnen und schließen. Eine solche Ausführungsform ist beispielsweise aus Metall gut herstellbar. Zwischen den Gehäuseteilen (3) und (5) sollte dann vorteilhafter weise eine Dichtung (10) eingelegt werden. Es hat sich gezeigt, dass hierfür eine Dichtungsmatte, die im Gehäuseteil (5) enthalten ist, besonders günstig ist. Eine solche Dichtungsmatte könnte beispielsweise aus Siliconmaterial bestehen.

Bei der erfindungsgemäßen Lösung fest miteinander verbundener Gehäuseteile (3) und (5), kann auch auf eine Öffnung (6) mit einem Verschluss (12) verzichtet werden, indem durch Aufbrechen der Gehäuseteile (3) und (5) an der Sollbruchlinien (13) die Sterilisationstestvorrichtung geöffnet wird und so der Indikator (2) entnommen werden kann.

Ebenfalls für fest miteinander verbundener Gehäuseteile (3) und (5) wird erfindungsgemäß vorgesehen, die Gehäuseteile(3) und (5) aus einem durchscheinenden Material herzustellen, um dabei ohne Öffnen oder Aufbrechen an einer Sollbruchlinien (13) den Indikator (2) beobachten bzw. dessen Reaktion feststellen zu können.

Eine weitere Ausgestaltung der Sterilisationstestvorrichtung sieht vor, dass diese aus mehr als zwei aufeinander abgestimmten Gehäuseteilen (3) und (5) bestehen. Hierbei handelt es sich um eine sog. mehrlagige Sterilisationstestvorrichtung. Mit dieser Ausführungsform ist die Möglichkeit gegeben Sterilisationstestvorrichtungen herzustellen, deren räumliches Ausmaß weniger in der Breite und Länge liegt als in deren Höhe.

In diesem Fall enthält der Gehäuseteil (3") dabei den Zuleitungsgraben (4) mit einer Öffnung (7), die eine Verbindung nach außen herstellt. Auch der Gehäuseteil (3') enthält einen Zuleitungsgraben (4), der mit einer Öffnung (7') versehen ist, die eine Verbindung zur Testkammer (1), mit einem darin eingelegten Indikator (2), ermöglicht. Beide Gehäuseteile (3') und (3") sind wiederum durch eine Öffnung (11) miteinander verbunden.

Bei den mehrlagigen Ausführungsformen sind erfindungsgemäß ebenfalls Mäander oder Schnecken sinnvoll, um gegebenenfalls die notwendige Länge des Zuleitungsgraben (4) in besonders kleinen Gehäusen (3) und (5) der Sterilisationstestvorrichtung unterbringen zu können. Für die mehrlagige Ausführungsform sind ebenfalls die weiter oben aufgeführten erfindungsgemäßen Lösungen anwendbar, die bereits für einlagig fest miteinander verbundener Gehäuseteile (3) und (5) vorgesehen und beschrieben sind.

Vorteilhafter Weise bestehen die Gehäuseteile (3) und (5) aus Kunststoff, wobei die Gehäuseteile auch jeweils aus unterschiedlichen Kunststoffmaterialien bestehen können. Dies ist immer dann sinnvoll, wenn dabei ein durchscheinendes Kunststoffmaterial Verwendung findet, um damit den Indikator (2) sichtbar werden zu lassen.

Es hat sich gezeigt, dass die Gehäuseteile(3) und (5) aus einem hitzebeständigen Kunststoff, die für ein Spritzgussverfahren geeignet sind, besonders günstige Materialien zur Herstellung der erfindungsgemäßen Sterilisationstestvorrichtung darstellen.

Alternativ zu dieser Lösung, bei der zur Entnahme des Indikators im Gehäuseteil (3) oder (5) eine Öffnung vorgesehen ist, die mit einem Einwegverschluss geschlossen werden muss, wird erfindungsgemäß eine fertigungstechnisch sehr effektive Variante vorgeschlagen, die eine quantitative Aussage über die Sterilisation ermöglicht.
Diese Variante der Sterilisationstestvorrichtung gestattet damit nicht nur eine Aussage ob der Sterilisationsprozess vollständig abgelaufen ist, sondern gibt vielmehr Auskunft über die Leistungsfähigkeit des Sterilisators.

Dies wird erfindungsgemäß dadurch gelöst, dass in dem Gehäuseteil (3) oder im Gehäuseteil (5) oder in beiden der Zuleitungsgraben (4) eingearbeitet sein kann, der zur Zuführung des Sterilisationsmediums dient.
Durch eine zumindest teilweise mäanderförmige und / oder schneckenförmige Anordnung des Zuleitungsgrabens bilden sich Stege im Inneren des Gehäuses aus, die zumindest teilweise mit dem /den Seitenteil(en) der Gehäuseteile (3) und / oder (5) einen Teil des Zuleitungsgrabens (4) bilden.
Ein so gebildeter Zuleitungsgraben (4) soll den Indikator (2) aufnehmen.

Die Erfindung sieht wahlweise auch vor, dass der Zuleitungsgraben derart angeordnet werden kann, dass sich durch diese Anordnung oder durch zumindest einen Teil eines Seitenteils des / der Gehäuseteile (3) und / oder (5) eine Testkammer (1) freigegeben wird, in der ein Indikator (2) eingebracht sein kann.

Der in das Gehäuseteil (3) und / oder (5) eingearbeitete Zuleitungsgraben (4) weist dabei einen beliebigen Querschnitt auf. Vorzugsweise kann dieser rechteckig oder quadratisch ausgebildet werden.

Durch das Einarbeiten des Zuleitungsgrabens in das Gehäuseteil wird eine sehr kompakte Bauweise realisiert. Der Zuleitungsgraben liegt geschützt im Inneren des Gehäuses und ist gegen äußere Beschädigungen optimal geschützt.

Nach einer bevorzugten Ausführungsvariante weist der Zuleitungsgraben (4) im Verhältnis zum Querschnitt eine solche große Länge auf, dass der Zuleitungsgraben (4) unter normalen Sterilisationsbedingungen nicht komplett entlüftet werden kann.

In diesem Fall macht es Sinn in den kompletten Zuleitungsgraben einen oder mehrere Indikatoren einzubringen, die exakt die Strecke anzeigen, bis zu der das Sterilisationsmedium vorgedrungen ist.
Derartige vorteilhafte Ausgestaltungen, bei denen wahlweise der Indikator (2) über die gesamte Läge des Zuleitungsgrabens oder mehrere Indikatoren (2) verteilt über die Länge des Zuleitungsgrabens (4) eingebracht sind, werden in den Unteransprüchen vorgeschlagen.

Diese Ausführung ist beispielsweise für periodische Tests des Sterilisators geeignet, da er im Gegensatz zu den herkömmlichen Sterilisationstestvorrichtungen eine quantitative Aussage über die maximal zu erreichende Strecke der Sterilisation beispielsweise eines Schlauches gleichen Durchmessers gibt und man Trends in der Leistungsfähigkeit des Sterilisators frühzeitig erkennen kann.
Hierzu macht es Sinn eine Art Skala auf das Gehäuse aufzubringen um die Werte sicher ablesen und dokumentieren zu können. In diesem Fall wird das Gehäuse aus einem durchscheinenden Material herzustellen sein, um das Ergebnis direkt ablesen zu können.
Bei dieser vorgenannten Ausführung ist es ebenso sinnvoll, dass die Sterilisationstestvorrichtung aus mehr als 2 aufeinander abgestimmten Gehäuseteilen (3) und (5) besteht. Hierbei handelt es sich dann um eine mehrlagige Sterilisationstestvorrichtung, deren räumliches Ausmaß weniger in der Breite und Länge liegt als in der Höhe.

Eine für den Einweggebrauch verwendete Sterilisationstestvorrichtung wird erfindungsgemäß mit einem maschinenlesbaren, fortlaufenden Code versehen, da so eine eindeutige Rückführbarkeit der Testsysteme bis hin zum Herstellungsprozess der Testvorrichtungen gewährleistet ist.

Vorteilhafter Weise bestehen die Gehäuseteile (3) und (5), ebenfalls aus Kunststoff, wobei die Gehäuseteile auch jeweils aus unterschiedlichen Kunststoffmaterialien bestehen können. Dies ist immer dann sinnvoll, wenn dabei ein durchscheinendes Kunststoffmaterial Verwendung findet, um damit den Indikator (2) durch die Gehäuseteile (3) und / oder (5) sichtbar werden zu lassen.
Es hat sich gezeigt, dass die Gehäuseteile (3) und (5) aus einem hitzebeständigem Kunststoff, die für ein Spritzgussverfahren geeignet sind, besonders günstige Materialien zur Herstellung der erfindungsgemäßen Sterilisationstestvorrichtung darstellen.

Wahlweise können die Gehäuseteile so gestaltet sein, dass sie die Möglichkeit bieten, sich sowohl vor als auch nach dem Sterilisationsvorgang öffnen und schließen zu lassen, um an den Indikator (2) zu gelangen oder die Gehäuseteile (3) und (5) sind fest miteinander verbunden.

Im letzteren Fall werden erfindungsgemäß der / die Steg(e) (9) und die Gehäuseteile (3) und (5) den Zuleitungsgraben (4) so ausgebildet, dass sie nach außen und zu sich selbst hin sterilisationsmediendicht abschließen.

In der Ausführungsform bei der die Gehäuseteile fest miteinander verbunden sind, werden wahlweise im Gehäuseteil (3) oder (5), eine Aufreißlasche (14) eingearbeitet, die es erlaubt, das Gehäuseteil (3) oder (5) durch aufreißen zu öffnen und den Indikator (2) zu entnehmen.

Eine weitere besonders bevorzugte Ausführungsvariante besteht für den Fall, dass das Gehäuseteil (3) oder (5), welches nicht den/ die Steg(e) (9) beinhaltet, eine Folie (16) darstellt.
Eine solche Lösung stellt dabei eine besonders kostengünstige Variante der Erfindung dar.

Der besondere Vorteil der erfindungsgemäßen Lösung besteht darin, eine sehr preisgünstige Sterilisationstestvorrichtungen herzustellen, da diese in sehr hoher Stückzahl produziert werden kann. Es besteht die Möglichkeit die Erfindung so auszugestalten, dass sie für den Einmal- oder Mehrfachgebrauch eingesetzt werden kann.

Ein besonderer Vorteil wird jedoch bei der Nutzung der Sterilisationstestvorrichtungen im Einmalgebrauch erzielt. Hierbei entsteht eine außerordentlich kompakte Vorrichtung, die vor äußerlichen Einwirkungen besonderen Schutz bietet. Da im Sterilisationsumfeld sehr viel auf Sicherheit Wert gelegt werden muss und insbesondere durch das Personal möglichst wenige Fehlerquellen vorhanden sein müssen, ist diese kompakte Lösung eine bevorzugte Ausführungsform. Mit der kompakten Sterilisationstestvorrichtung wird größtmögliche Sicherheit dadurch erzielt, dass durch das Krankenhauspersonal keine Beeinflussung der Testsvorrichtung vor und nach dem Sterilisationsvorgang durch das öffnen und Schließen möglich ist.

Das Personal braucht hierzu nur die Sterilisationstestvorrichtungen in den Sterilisation Automaten einlegen und nach den Sterilisationszyklus entweder die Einmalöffnung zum Zwecke der Entnahme des Indikators betätigen oder das Ergebnis der Sterilisation über die durchsichtige Gehäusehälfte am Indikator ablesen.

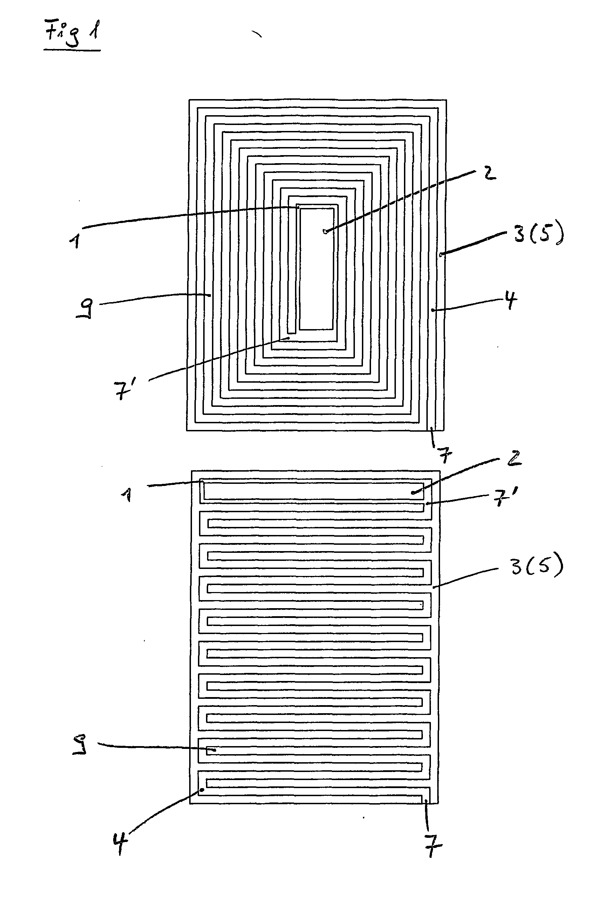

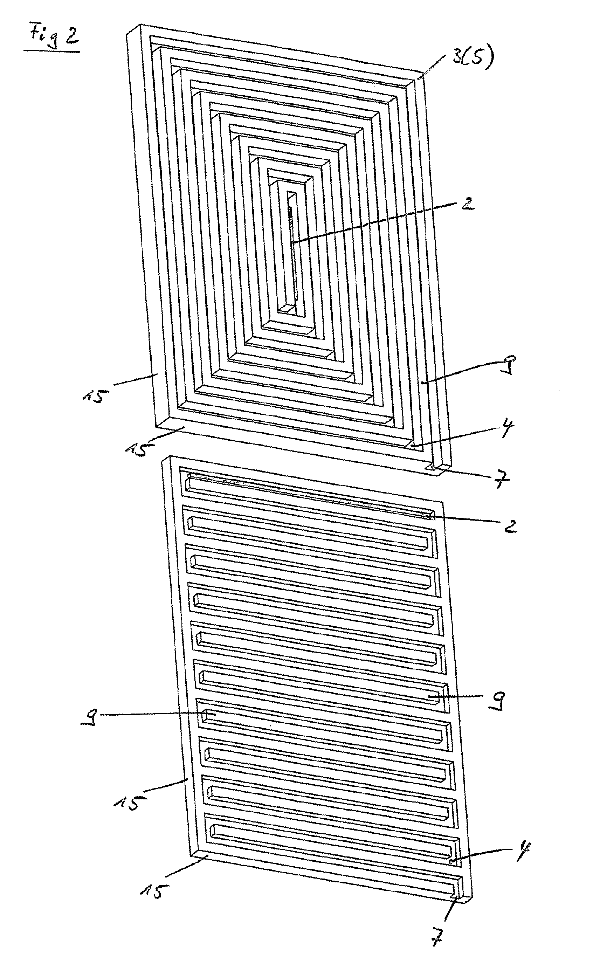

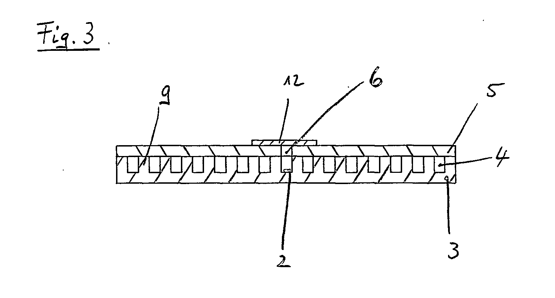

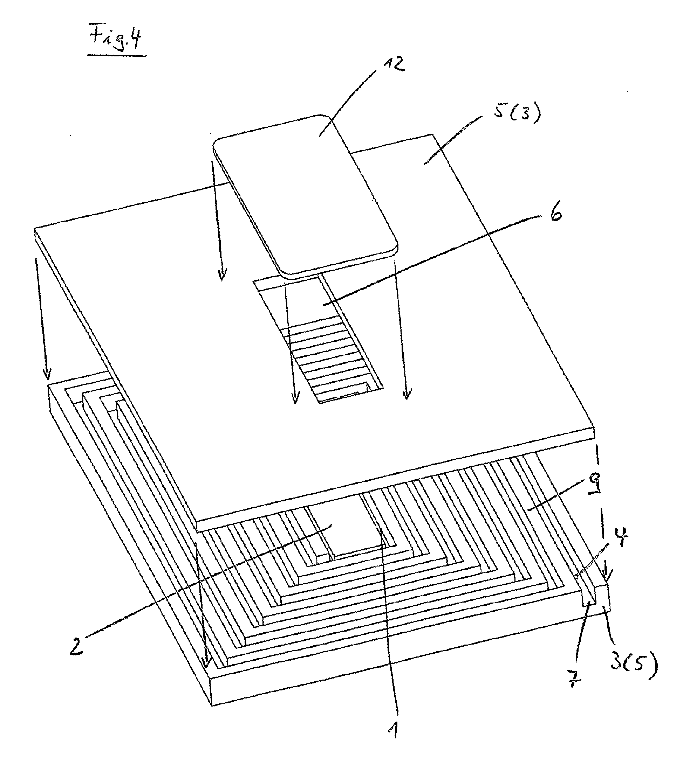

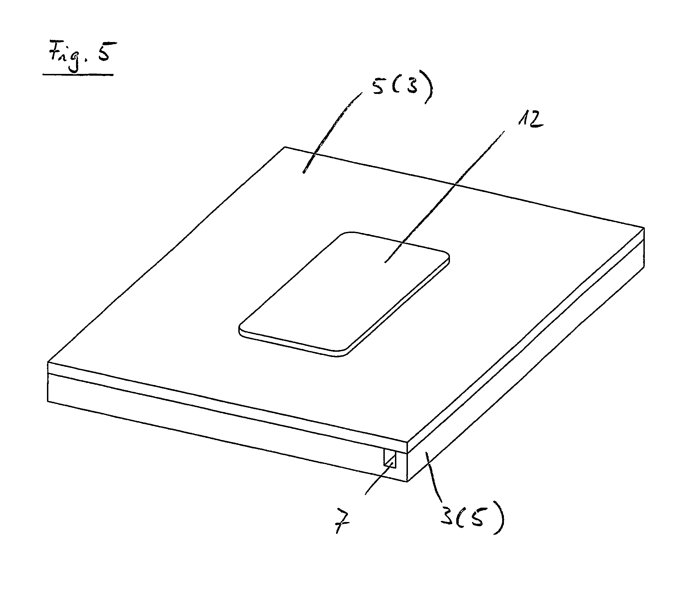

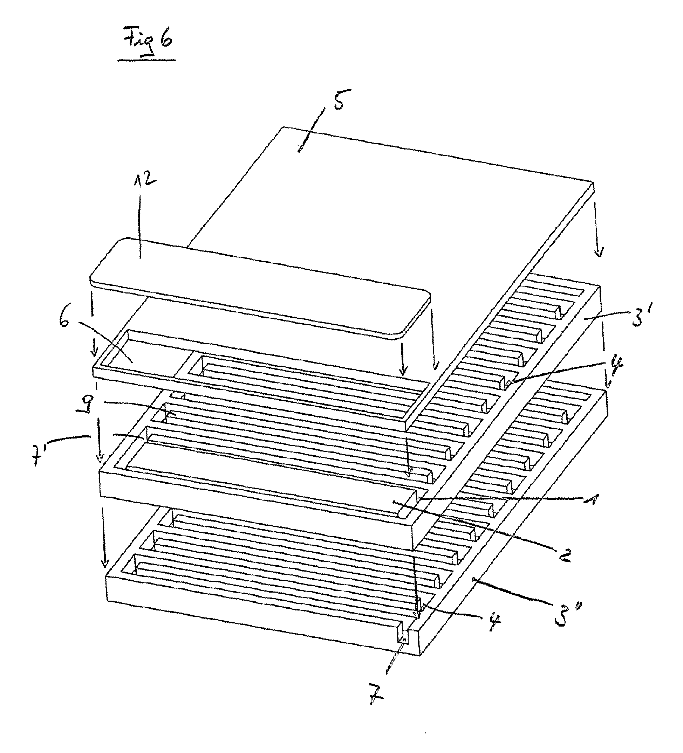

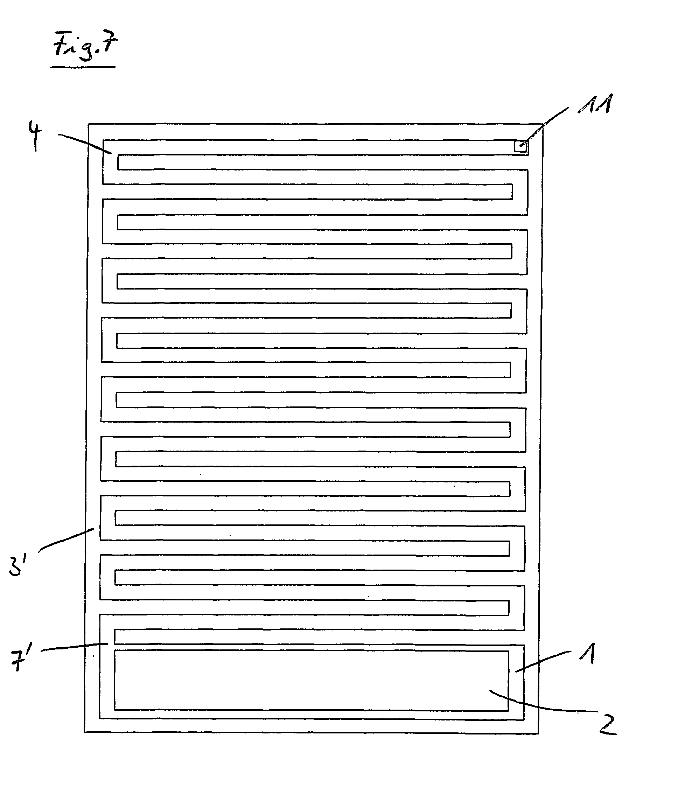

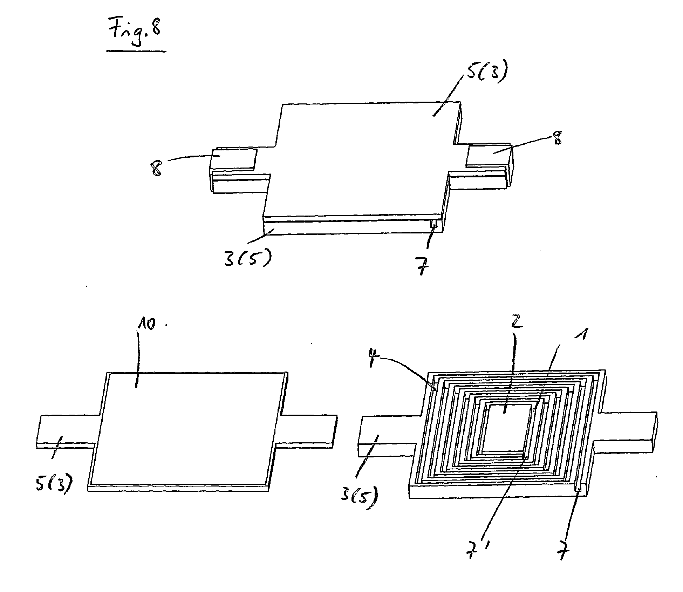

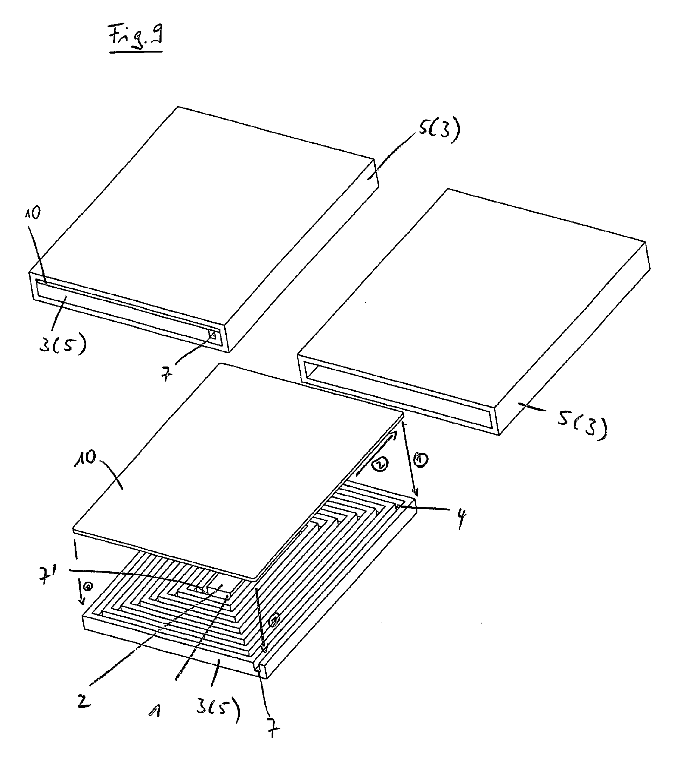

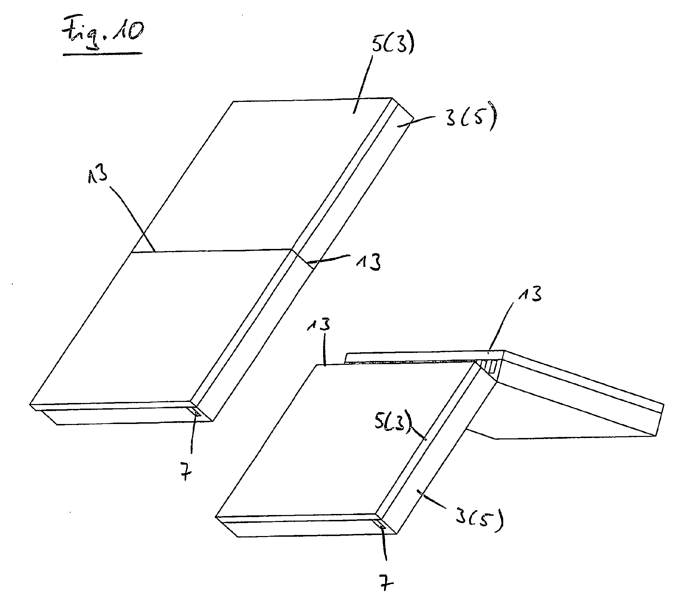

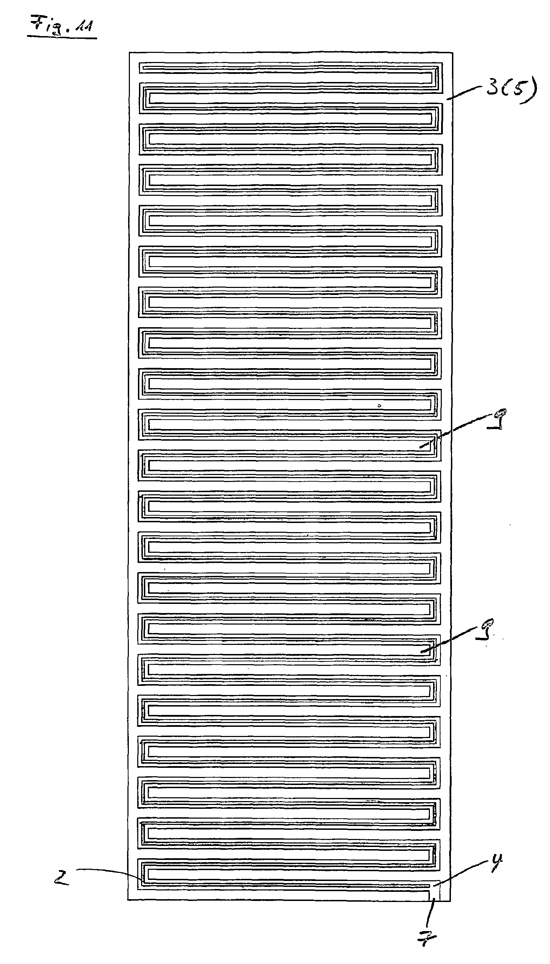

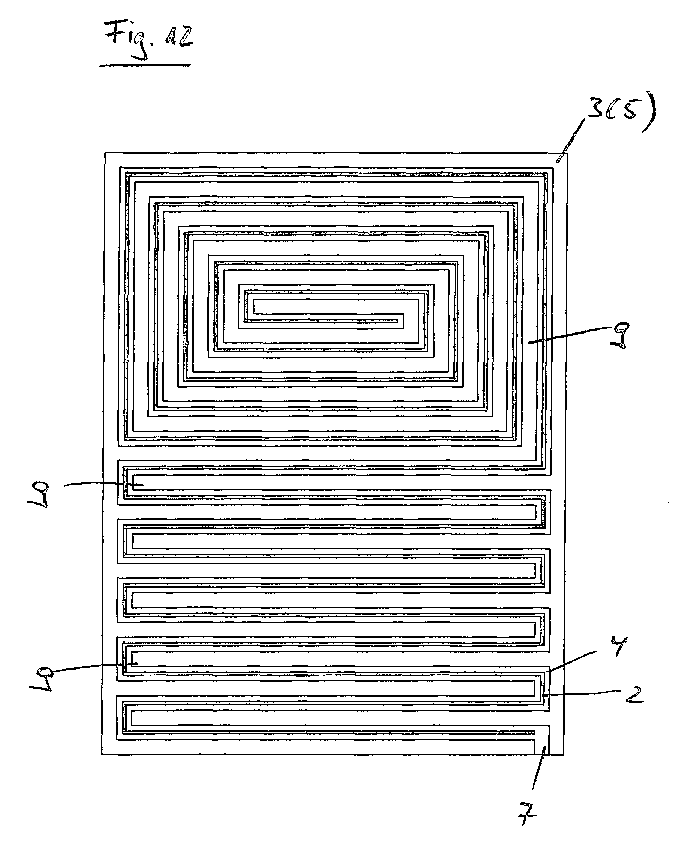

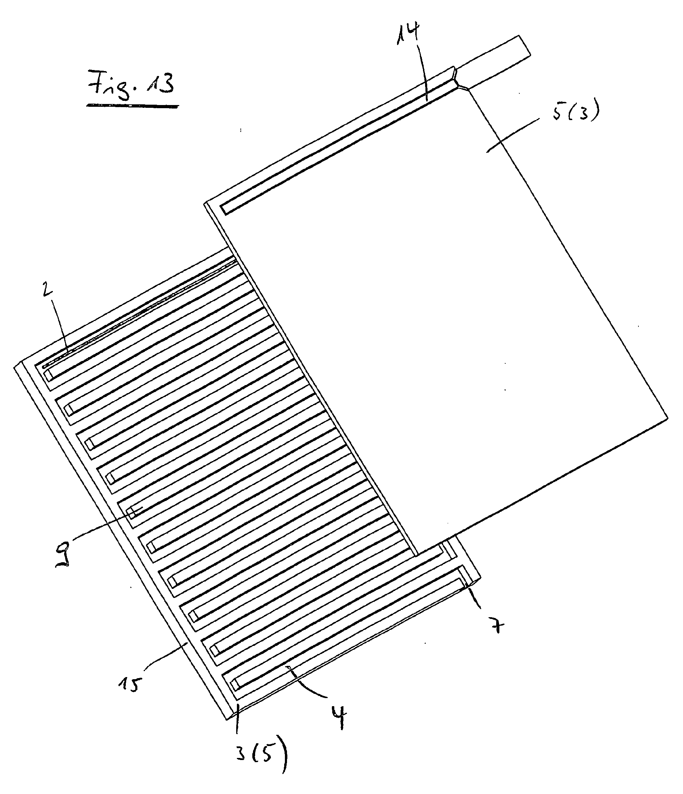

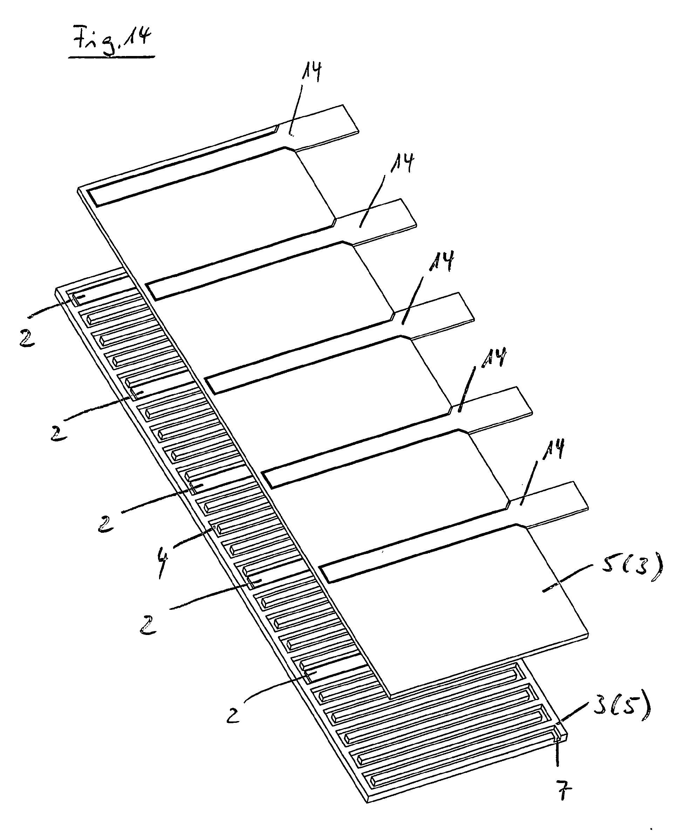

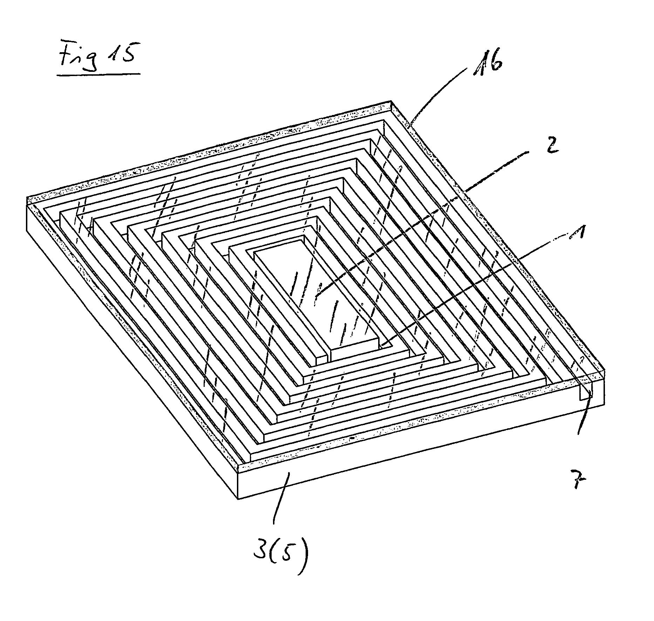

Zur näheren Erläuterung wird die erfindungsgemäße Lösung anhand von Zeichnungen in Fig.1 bis Fig. 15 dargestellt.

Es zeigen:
[Fig.1] Das Gehäuseteil (3) / (5) in der Draufsicht ohne entsprechendes Gehäuseteil (5) / (3) mit schneckenförmigen bzw. mäanderförmigen Zuleitungsgraben (4), ausgebildeter Testkammer (1) mit eingebrachtem Indikator (2)
In [Fig.2] wird ein Gehäuseteil (3) oder (5) dargestellt, in das einmal ein schneckenförmiger und einmal ein mäanderförmiger Zuleitungsgraben (4) eingearbeitet ist und sich durch diesen Zuleitungsgraben (4) zumindest ein Steg (9) ausbildet. In dieser Darstellung sieht man auch sehr gut wie sich durch die Gehäuseseitenteile (15) und den / die Steg(e) (9) der Zuleitungsgraben (4) darstellt.
[Fig.3] zeigt eine Schnittdarstellung einer Sterilisationstestvorrichtung mit einem schneckenförmig eingearbeiteten Zuleitungsgraben (4) in das Gehäuse (3) das durch ein Gehäuseteil (5) fest miteinander verbunden ist und das Gehäuseteil (5) eine Öffnung (6) aufweist, die durch einen Verschluss (12) geschlossen ist. Der Indikator (2) ist direkt in den Zuleitungsgraben (4) eingebracht.
[Fig.4] zeigt eine Explosionsdarstellung einer Sterilisationstestvorrichtung mit schneckenförmigem Zuleitungsgraben (4) im Gehäuseteil (3) bzw. (5). Das Gehäuseteil (5) bzw. (3) weist eine Öffnung (6) auf, die mit einem Verschluss (12) verschlossen ist. Durch Öffnen des Verschlusses (12) gelangt man an den Indikator (2) in der Testkammer (1).
[Fig.5] zeigt [Fig.4] als Zusammenbau.
[Fig.6] zeigt eine mehrlagige Sterilisationstestvorrichtung.
[Fig. 7] zeigt in der Draufsicht das Gehäuseteil 3' mit Öffnung (11)
[Fig. 8] eine Variante einer Mehrwegsterilisationstestvorrichtung, bei der ein unbeabsichtigtes Öffnen der Gehäuseteile (3) und (5) durch Klammern (8) verhindert wird.
[Fig.9] eine Variante einer Mehrwegsterilisationstestvorrichtung, wobei das Gehäuseteil (3) sowie die Dichtung (10) in das Gehäuseteil (5) eingeschoben wird.
[Fig.10] eine Einwegsterilisationstestvorrichtung, bei der zum Entnehmen des Indikators (2) die Gehäuseteile (3) und (5) an der Sollbruchlinie (13) aufgebrochen werden.
[Fig.11] zeigt eine Sterilisationstestvorrichtung bei der schematisch dargestellt sein soll, das ein zum Querschnitt sehr langer Zuleitungsgraben (4) eingearbeitet ist, der unter normalen Sterilisationsbedingungen nicht komplett entlüftet werden kann. In diesem Fall ist der Indikator (2) über die komplette Länge des Zuleitungsgraben (4) eingebracht um zu überprüfen in wieweit das Sterilisationsmedium in dem Zuleitungsgraben (4) vorgedrungen ist.
[Fig. 12] zeigt eine teilweise mäander- und schneckenförmige Ausgestaltung des Zuleitungsgrabens (4). Der Indikator (2) ist hier in dem kompletten Zuleitungsgraben (4) eingebracht.
[Fig. 13] wird anhand einer Explosionsdarstellung ,gezeigt, wie durch eine Aufreißlasche (14) das Gehäuseteil (5) bzw.(3) geöffnet werden kann um den Indikator (2) entfernen zu können.
[Fig.14] zeigt eine Explosionsdarstellung einer Sterilisationstestvorrichtung bei der in das Gehäuseteil (3) oder (5) mehrere Indikatoren (2) eingebracht sind. Durch Öffnen der Aufreißlaschen (14) können sie einzelnen Indikatoren (14) entnommen werden.
[Fig.15] zeigt eine bevorzugte Ausführungsvariante bei der im Gehäuseteil (3) oder (5) der Zuleitungsgraben (4) eingearbeitet ist und das Gehäuseteil (5) oder (3) mit einer transparenten Folie (16) abgedichtet ist.

Darin bedeuten:
- (1): Testkammer
- (2): Indikator
- (3): unteres Gehäuseteil
- (3'): Gehäuseteil bei mehrlagiger Ausführung
- (3"): Gehäuseteil bei mehrlagiger Ausführung
- (4): Zuleitungsgraben
- (5): oberes Gehäuseteil
- (6): Öffnung im Gehäuseteil (3), (5)
- (7): Öffnung am Zuleitungsgraben (4)
- (7'): Öffnung am Zuleitungsgraben (4) zur Testkammer (1)
- (8): Klammern
- (9): Steg(e)
- (10): Dichtung
- (11): Öffnung zwischen (3') und (3")
- (12): Verschluss zu (6)
- (13): Sollbruchlinie
- (14): Aufreißlasche
- (15): Gehäuseseitenteil.
- (16): Folie

Die Erfindung wird nachfolgend an einigen Ausführungsbeispielen noch näher erläutert.

Eine bevorzugte Ausführungsformen bestehet darin, dass die Gehäuseteile (3) und (5) aus einem hitze- und mechanisch beständigem Kunststoffmaterial und werden im Spritzgussverfahren hergestellt. Das Gehäuseteil (5) weist eine Öffnung (6) auf, die mit einem Einwegverschluss (12) geschlossen ist. Der Einwegverschluss (12) besteht aus einer mechanisch und hitzebeständigen Folie, die bei der Herstellung gasdicht aufgeprägt wird und somit den Zugang zur Testkammer (1) verschließt.

Die Gehäuseteile (3) und (5) werden in Herstellungsprozess nach dem Einlegen des Indikators (2) in die Testkammer (1) durch Ultraschallschweißen unlösbar miteinander verbunden.

Das Öffnen der Sterilisationstestvorrichtung, um den Indikator (2) nach durchgeführter Sterilisation aus der Testkammer (1) entnehmen zu können, erfolgt über ein Abreißen der Folie.

Eine weitere bevorzugte Ausführungsvariante der Erfindung sieht vor, das im Gehäuseteil (3) oder (5) der Zuleitungsgraben (4) derart angeordnet ist, das der Zuleitungsgraben (4) nach einer bestimmten Länge in eine Testkammer (1) mündet. Danach setzt sich der Zuleitungsgraben fort und mündet in die nächste Testkammer. Dieses System aus der Aneinanderreihung von Zuleitungsgraben und Testkammer sollte so lange ausgestaltet sein, das es sich unter normalen Sterilisationsbedingungen nicht komplett entlüften lässt. Das Gehäuseteil (3) oder (5) lässt sich über die Aufreißlaschen öffnen um an die Indikatoren zu gelangen. Somit lässt sich bestimmen, bis zu welcher Länge sich der Zuleitungsgraben entlüften ließ.

Dadurch, dass es sich bei der bevorzugten Ausgestaltung um einen Einwegartikel handelt, hat sich gezeigt, dass es von Vorteil ist, den Einwegartikel über eine Seriennummer eindeutig identifizieren zu können. Ebenfalls als vorteilhaft hat sich gezeigt, die Seriennummer als Barcode zu verschlüsseln.

Bei einer weiteren vorteilhaften Ausgestaltung der Erfindung ist im Gehäuseteil (3) oder (5) der Zuleitungsgraben (4) eingearbeitet. Der Zuleitungsgraben mündet in eine Testkammer in die ein Indikator eingebracht ist. Das Gehäuseteil (3) oder (5) ist mit einer transparenten Folie abgedichtet, so dass die Reaktion des Indikators durch die Folie begutachtet werden kann und durch ablösen der Folie der Indikator zugänglich ist.

## Patentansprüche

1. Sterilisationstestvorrichtung aus mindestens zwei Gehäuseteilen (3,5) und einer Testkammer (1) mit Indikator (2), mit Öffnungen und Zuleitungen zur Zuführung des Sterilisationsmediums,
**dadurch gekennzeichnet, dass**
in einem Gehäuseteil (3,5), der aufeinander abgestimmten Gehäuseteile (3,5), ein Zuleitungsgraben (4) so eingegraben ist, dass sich durch dessen Anordnung im Inneren des Gehäuseteiles (3,5) ein oder mehrere Steg/Stege (9) ausbildet/ausbilden, zwischen dem/denen der Zuleitungsgraben (4) liegt, am äußeren Rand des Gehäuseteiles (3,5) der (die) Steg (e) zumindest teilweise mit einem Seitenteil (15) der Gehäuseteile (3,5) den Zuleitungsgraben (4) bildet (n), der / die Steg (e) (9) und die Gehäuseteile (3,5) den Zuleitungsgraben (4) nach außen und zu sich selbst hin sterilisationsmediendicht abschließt (en), der Zuleitungsgraben (4) auch den Grundkörper der Testkammer (1) bildet, in der der Indikator (2) eingelegt ist und das Sterilisationsmedium von außen direkt über die Öffnung (7) in den Zuleitungsgraben (4) und zum Indikator (2) gelangt.

2. Sterilisationstestvorrichtung nach Anspruch 1, **dadurch gekennzeichnet, dass** der Indikator (2) über die ganze Länge des Zuleitungsgrabens (4) eingebracht ist.

3. Sterilisationstestvorrichtung nach Anspruch 1, **dadurch gekennzeichnet, dass** mehrere Indikatoren (2) verteilt über die Länge des Zuleitungsgrabens (4) eingebracht sind.

4. Sterilisationstestvorrichtung nach Anspruch 1, **dadurch gekennzeichnet, dass** der Zuleitungsgraben (4) einen quadratischen oder rechteckigen Querschnitt aufweist.

5. Sterilisationstestvorrichtung nach Anspruch 1, **dadurch gekennzeichnet, dass** der Zuleitungsgraben (4) als Mäander und / oder Schnecke ausgebildet ist.

6. Sterilisationstestvorrichtung nach Anspruch 1, **dadurch gekennzeichnet, dass** die Gehäuseteile (3) und (5) fest miteinander verbunden sind.

7. Sterilisationstestvorrichtung nach Anspruch 6, **dadurch gekennzeichnet, dass** das Gehäuseteil (3) oder (5), welches nicht den / die Steg (e) beinhaltet, eine Folie (16) darstellt.

8. Sterilisationstestvorrichtung nach Anspruch 6, **dadurch gekennzeichnet, dass** die Gehäuseteile (3) und / oder (5) aus einem durchscheinenden Material bestehen.

9. Sterilisationstestvorrichtung nach Anspruch 6, **dadurch gekennzeichnet, dass** die Gehäuseteile (3) und (5) eine Sollbruchlinie (13) aufweisen.

10. Sterilisationstestvorrichtung nach Anspruch 6, **dadurch gekennzeichnet, dass** in dem Gehäuseteil (3) oder (5) mindestens eine Aufreißlasche (14) eingearbeitet ist.

11. Sterilisationstestvorrichtung nach Anspruch 6, **dadurch gekennzeichnet, dass** die Gehäuseteile (3) oder (5) eine Öffnung (6) aufweisen, die mittels eines Verschlusses (12) geschlossen ist.

12. Sterilisationstestvorrichtung nach Anspruch 11, **dadurch gekennzeichnet, dass** der Verschluss (12) als Einwegverschluss ausgebildet ist.

13. Sterilisationstestvorrichtung nach Anspruch 1, **dadurch gekennzeichnet, dass** auf dem Gehäuseteil (3) und / oder (5) eine Seriennummer aufgebracht ist.

14. Sterilisationstestvorrichtung nach Anspruch 13, **dadurch gekennzeichnet, dass** die Seriennummer als bar- oder Strichcode aufgebracht ist.

15. Sterilisationstestvorrichtung nach Anspruch 2 und 3, **dadurch gekennzeichnet, dass** das Gehäuseteil (3) und /oder (5) mit einer Skala versehen ist.

16. Sterilisationstestvorrichtung nach Anspruch 1, **dadurch gekennzeichnet, dass** bei mehrlagiger Ausführung die Gehäuseteile (3') und (3") durch eine Öffnung (11) miteinander verbunden sind, das Gehäuseteil (3") den Zuleitungsgraben (4) mit der Öffnung (7) nach außen aufweist und das Gehäuseteil (3') den Zuleitungsgraben (4) mit der Öffnung (7') zu der Testkammer (1), mit dem eingelegten Indikator (2), enthält.

17. Sterilisationstestvorrichtung nach Anspruch 1, **dadurch gekennzeichnet, dass** die Gehäuseteile (3) und (5) lösbar miteinander verbunden sind und zwischen den Gehäuseteile (3) und (5) eine Dichtung (10) enthalten.

18. Sterilisationstestvorrichtung nach Anspruch 17, **dadurch gekennzeichnet, dass** im Gehäuseteil (3) oder (5) als Dichtung (10) eine Dichtungsmatte eingelegt ist

19. Sterilisationstestvorrichtung nach Anspruch 1, **dadurch gekennzeichnet, dass** die Gehäuseteile (3) und (5) aus einem hitzebeständigen Kunststoffmaterial bestehen.

20. Sterilisationstestvorrichtung nach Anspruch 19, **dadurch gekennzeichnet, dass** die Gehäuseteile (3) und (5) jeweils aus verschiedenen Kunststoffmaterialien bestehen.

21. Sterilisationstestvorrichtung nach Anspruch 19, **dadurch gekennzeichnet, dass** die hitzebeständigen Kunststoffmaterialien für ein Spritzgussverfahren geeignet sind.

## Claims

1. Sterilisation testing device consisting of at least two housing parts (3,5) and a test chamber (1) with indicator (2), with openings and supply lines for the supply of the sterilisation medium, **characterized in that** in one housing part (3,5), of the matching housing parts (3,5), a supply line channel (4) has been worked in such a manner that its arrangement in the inside of the housing part (3,5) forms one or several web/s (9) between which the supply line channel (4) lies, at the outer edge of the housing part (3,5) which forms the web(s), at least partially, with one lateral part (15), the housing parts (3,5), the supply line channel (4), closing the web(e) (9) and the housing parts (3,5), the supply line channel (4) towards the outside and itself in a manner leakproof for the sterilisation medium, forming the supply line channel (4), also the base body of the test chamber (1) in which the indicator (2) is placed and where the sterilisation medium reaches the indicator (2) from the outside directly through the opening (7) in the supply line channel (4).

2. Sterilisation testing device according to claim 1, **characterized in that** the indicator (2) is placed inside over the entire length of the supply line channel (4).

3. Sterilisation testing device according to claim 1, **characterized in that** several indicators (2) are placed inside spread over the length of the supply line channel (4).

4. Sterilisation testing device according to claim 1, **characterized in that** the supply line channel (4) exhibits a square or rectangular cross-section.

5. Sterilisation testing device according to claim 1, **characterized in that** the supply line channel (4) is fashioned as meander and / or helix.

6. Sterilisation testing device according to claim 1, **characterized in that** the housing parts (3) and (5) are firmly joined together.

7. Sterilisation testing device according to claim 6, **characterized in that** the housing part (3) or (5) which does not contain the web(s) represents a foil (16).

8. Sterilisation testing device according to claim 6, **characterized in that** the housing parts (3) and / or (5) consist of a translucent material.

9. Sterilisation testing device according to claim 6, **characterized in that** the housing parts (3) and (5) exhibit a preset breaking line (13).

10. Sterilisation testing device according to claim 6, **characterized in that** at least one tear strip (14) has been integrated in the housing part (3) or (5).

11. Sterilisation testing device according to claim 6, **characterized in that** the housing parts (3) or (5) have an opening (6) that is closed by a closure (12).

12. Sterilisation testing device according to claim 11, **characterized in that** the closure (12) is a one-way closure.

13. Sterilisation testing device according to claim 1, **characterized in that** a serial number has been affixed to the housing parts (3) and / or (5).

14. Sterilisation testing device according to claim 13, **characterized in that** the serial number is affixed as bar or line code.

15. Sterilisation testing device according to claims 2 and 3, **characterized in that** the housing part (3) and / or (5) bears a scale.

16. Sterilisation testing device according to claim 1, **characterized in that** with multi-layer design the housing parts (3') and (3") are joined by an opening (11), the housing part (3") exhibits the supply line channel (4) with the opening (7) toward the outside and that the housing part (3') contains the supply line channel (4) with the opening (7') toward the testing chamber (1) with inserted indicator (2).

17. Sterilisation testing device according to claim 1, **characterized in that** the housing parts (3) and (5) are separably joined together and that a seal (10) is placed between the housing parts (3) and (5).

18. Sterilisation testing device according to claim 17, **characterized in that** a sealing mat has been placed as seal into housing part (3) or (5).

19. Sterilisation testing device according to claim 1, **characterized in that** the housing parts (3) and (5) consist of a heat-resistant plastic material.

20. Sterilisation testing device according to claim 19, **characterized in that** the housing parts (3) and (5) each consist of different plastic materials.

21. Sterilisation testing device according to claim 19, **characterized in that** the heat-resistant plastic materials are suitable for an injection molding process.

## Revendications

1. Dispositif de test de stérilisation, composé au minimum de deux parties de boîtier (3,5) et d'une chambre de test (1) pourvue d'un indicateur (2), d'ouvertures et de conduites d'alimentation du milieu de stérilisation, **caractérisé en ce qu'**une rainure d'alimentation (4) est creusée dans une des parties du boîtier (3,5) adaptées les unes aux autres (3,5), de façon à ce que sa disposition à l'intérieur de la partie du boîtier (3,5) forme une ou plusieurs entretoise/s (9), entre lesquelles se situe la rainure d'alimentation (4), que, sur le bord extérieur de la partie du boîtier (3,5), la/les entretoise/s constitue/nt, au moins en partie, la rainure d'alimentation (4) avec une partie latérale (15) de la partie du boîtier der (3,5), que la/les entretoise/s (9) et les parties du boîtier (3,5) ferme/nt la rainure d'alimentation (4) vers l'extérieur et vers elles-mêmes de façon étanche au milieu de stérilisation, que la rainure d'alimentation (4) constitue également le corps principal de la chambre de test (1) qui comporte l'indicateur (2), et que le milieu de stérilisation arrive directement de l'extérieur via l'ouverture (7) dans la rainure d'alimentation (4) et vers l'indicateur (2).

2. Dispositif de test de stérilisation selon la revendication 1, **caractérisé en ce que** l'indicateur (2) est inséré sur l'ensemble de la longueur de la rainure d'alimentation (4).

3. Dispositif de test de stérilisation selon la revendication 1, **caractérisé en ce que** plusieurs indicateurs (2) sont repartis sur la longueur de la rainure d'alimentation (4).

4. Dispositif de test de stérilisation selon la revendication 1, **caractérisé en ce que** la rainure d'alimentation (4) présente une section rectangulaire ou carrée.

5. Dispositif de test de stérilisation selon la revendication 1, **caractérisé en ce que** la rainure d'alimentation (4) présente une forme de méandre et/ou de colimaçon.

6. Dispositif de test de stérilisation selon la revendication 1, **caractérisé en ce que** les parties du boîtier (3) et (5) sont raccordées fermement.

7. Dispositif de test de stérilisation selon la revendication 6, **caractérisé en ce que** la partie du boîtier (3) ou (5) qui ne comporte pas l'entretoise/les entretoises, constitue un film (16).

8. Dispositif de test de stérilisation selon la revendication 6, **caractérisé en ce que** les parties du boîtier (3) et/ou (5) sont constituées par une matière transparente.

9. Dispositif de test de stérilisation selon la revendication 6, **caractérisé en ce que** les parties du boîtier (3) et (5) présentent une ligne de rupture intentionnelle (13).

10. Dispositif de test de stérilisation selon la revendication 6, **caractérisé en ce que** la partie du boîtier (3) ou (5) comporte au minimum une patte de déchirage (14).

11. Dispositif de test de stérilisation selon la revendication 6, **caractérisé en ce que** les parties du boîtier (3) ou (5) présentent une ouverture (6) obturée par une fermeture (12).

12. Dispositif de test de stérilisation selon la revendication 11, **caractérisé en ce que** la fermeture (12) ne peut être utilisée qu'une seule fois.

13. Dispositif de test de stérilisation selon la revendication 1, **caractérisé en ce que** la partie du boîtier (3) et/ou (5) comporte un numéro de série.

14. Dispositif de test de stérilisation selon la revendication 13, **caractérisé en ce que** le numéro de série est appliqué sous la forme d'un code à barres.

15. Dispositif de test de stérilisation selon les revendications 2 et 3, **caractérisé en ce que** la partie du boîtier (3) et/ou (5) est pourvue d'un cadran.

16. Dispositif de test de stérilisation selon la revendication 1, **caractérisé en ce que**, en version de plusieurs couches, les parties du boîtier (3') et (3") sont liées par une ouverture (11), que la partie du boîtier (3") présente la rainure d'alimentation (4) avec l'ouverture (7) orientée vers l'extérieur, et que la partie du boîtier (3') comporte la rainure d'alimentation (4) avec l'ouverture (7') orientée vers la chambre de test (1), où l'indicateur (2) est inséré.

17. Dispositif de test de stérilisation selon la revendication 1, **caractérisé en ce que** les parties du boîtier (3) et (5) sont raccordées de façon démontable, et qu'un joint d'étanchéité (10) est inséré entre les parties du boîtier (3) et (5).

18. Dispositif de test de stérilisation selon la revendication 17, **caractérisé en ce que** un matelas d'étanchéité (10) est inséré dans la partie du boîtier (3) ou (5).

19. Dispositif de test de stérilisation selon la revendication 1, **caractérisé en ce que** les parties du boîtier (3) et (5) sont fabriquées d'une matière plastique résistante à la chaleur.

20. Dispositif de test de stérilisation selon la revendication 19, **caractérisé en ce que** les parties du boîtier (3) et (5) sont fabriquées de différentes matières plastiques.

21. Dispositif de test de stérilisation selon la revendication 19, **caractérisé en ce que** les matières plastiques résistantes à la chaleur conviennent au procédé de moulage par injection.
